# EUROPEAN PATENT SPECIFICATION

(11) **EP 0 762 900 B1**
(45) Date of publication and mention of the grant of the patent: **21.11.2001**
(21) Application number: 95924646.3
(22) Date of filing: 21.06.1995
(51) Int. Cl.: A61K 49/04, C07C 233/05

(54) **IMPROVED SYNTHESIS OF IOVERSOL USING PHOSPHORIC ACID MODIFIED CO-ADDITION**
VERBESSERTE SYNTHESE VON IOVERSOL UNTER VERWENDUNG EINER MIT PHOSPHORSÄURE MODIFIZIERTEN KOADDITION
SYNTHESE AMELIOREE DE IOVERSOL PAR CO-ADDITION MODIFIEE D'ACIDE PHOSPHORIQUE

(30) Priority: 21.06.1994 US 263119
(43) Date of publication of application: 19.03.1997
(73) Proprietor: MALLINCKRODT INC., St. Louis, MO 63134 (US)
(72) Inventor: LEIBNER, James E., Maryland Heights, MO 63043 (US)
(74) Representative: Jones, Alan John
(86) International application number: US9507906
(87) International publication number: WO9535122

(56) References cited:
- JP-A- 3 167 153
- US-A- 3 988 369
- US-A- 5 013 865
- INDIAN JOURNAL OF CHEMISTRY, Volume 20A, issued February 1981, RAO et al., "Kinetics & Mechanism of Iodination of Phenol & Substituted Phenols by Iodine Monochloride in Aqueous Acetic Acid", pages 133-135.

## Description

### Field of the Invention

The present invention relates to a process for the preparation of N,N'-bis(2,3-dihydroxypropyl)-5-[N-(2-hydroxyethyl)glycolamido]-2,4,6-triiodoisophthalamide, hereinafter referred to by its generic name, ioversol. More particularly, this invention relates to an alternate co-addition process to increase the purity of an iodinated product compound such as an iodinated X-ray contrast agent (ioversol) for parenteral use.

### Background of the Invention

Ioversol is disclosed as a useful nonionic x-ray contrast agent in U.S. Patent Number 4,396,598. The co-addition process, which is one portion of a multi-step process whereby iodine is introduced into an intermediate molecule used in the production of ioversol, is disclosed in U.S. Patent Number 5,013,865. However, the co-addition process described in U.S. Patent Number 5,013,865 in practice is difficult to control and suffers from the formation of undesirable and difficult to remove colored by-products. It is possible to remove the colored by-products in subsequent purification steps but is time consuming and expensive.

Accordingly, an alternate iodination method that would eliminate color formation, increase purity, save time and decrease expense is desired. It is an object of the present invention to meet this need.

### Summary of the Invention

During the production of an iodinated product compound such as the iodinated X-ray contrast agent ioversol, the process comprises reaction of a substrate selected from among 5-amino-N-alkylisophthalamic acid, salts thereof, esters thereof, 5-aminoisophthalamide, 5-amino-N-(hydroxyalkyl)isophthalamide and 5-amino-N,N'-bis hydroxyalkylisophthalamide such as 5-amino-N,N'-bis(2,3-dihydroxypropyl)isophthalamide, with an iodine halide such as iodine monochloride solution or aqueous sodium dichloroiodate in an aqueous reaction medium such as water or a very dilute acid. The substrate and a source of the iodine halide are added to the reaction medium at rates such that at any instant during the addition cycle, the substrate is present in a stoichiometric excess of about 0 to 10 percent but preferably about 7 percent over that of the iodine halide. The cumulative amount of the iodine halide may be in stoichiometric excess of about 0 to 10 percent but preferably about 4 percent over that of the substrate. The addition process is further carried out in the presence of an alkaline buffer composition which is sufficient to maintain the reaction medium at a pH between about 0 and about 3 during the course of the reaction but at the beginning of the reaction at a pH of about 2.5 to 3.0. The addition process also requires a sufficient water volume such that the concentration of the iodinated product compound does not exceed about 0.08 moles/liter in the reaction mass at the conclusion of the iodination reaction. This standard co-addition process is described in detail in U.S. Patent No. 5,013,865.

The novel process of the present invention requires the addition of phosphoric acid or phosphoric acid and a salt thereof in the initial water charge of the co-addition process of the substrate such as those noted above and iodine halide used to produce an iodinated product compound, e.g., 5-amino-N,N'-bis(2,3-dihydroxypropyl)-2,4,6-triiodoisophthalamide. The substrate and a source of the iodine halide such as iodine monochloride or aqueous sodium dichloroiodate are added to the reaction medium at rates such that at any instant during the addition cycle, the substrate is present in a stoichiometric excess of about 0 to 50 percent but preferably within the range of about 7 to 15 percent over that of the iodine halide. The cumulative amount of the iodine halide may be present in a stoichiometric excess of about 0 to 10 percent but preferably about 1 percent over that of the substrate in an aqueous reaction medium such as water or a very dilute acid. The addition process is further carried out in the presence of an alkaline buffer composition which with the addition of phosphoric acid maintains the reaction medium at a pH between 1 and 2 from beginning to end. The process of the present invention likewise requires a sufficient water volume such that the concentration of the iodinated product compound does not exceed about 0.20 moles/liter but preferably between 0.10 and 0.20 moles/liter in the reaction mass at the conclusion of the iodination reaction. These modifications in the co-addition step of the production process surprisingly have been shown to be beneficial in reducing the level of colored reaction side products such that the color of the mother liquor typically improves from a deep red to a yellow color. This improvement in the process corresponds to reduced levels of the colored reaction side product azo-bis(5,5'-N,N'-bis(2,3-dihydroxypropyl)-2,4,6-triiodo)-1,3-benzenedicarboxamide.

The PAM co-addition process of the present invention whereby phosphoric acid or phosphoric acid and a salt thereof is used in the initial water charge of the co-addition process and used in conjunction with improved agitation methods significantly reduces the formation of the azo-bis(5,5'-N,N'-bis(2,3-dihydroxypropyl)-2,4,6-triiodo)-1,3-benzenedicarboxamide impurity in the iodination processing step. The ultimate result is an improved coloration of ioversol. The PAM co-addition process used in conjunction with or without modified agitation methods likewise improves yields, eliminates or reduces manufacturing expense associated with additional purification steps, improves attendant safety, and provides improved process throughput than the previously used standard co-addition process.

### Detailed Description of the Invention

N,N'-bis (2,3-dihydroxypropyl)-5-[N-(2-hydroxyethyl)glycolamido] -2,4,6-triiodoisophthalamide, i.e. ioversol, as disclosed in U.S. Patent Number 4,396,598 discussed above, may be prepared in accordance with the present invention by altering the previously disclosed standard co-addition process. The PAM co-addition process of the present invention includes the addition of enough phosphoric acid or phosphoric acid and a salt thereof to reach a pH within the range of 1.0 to 2.0 in the initial water charge of the co-addition step referred to herein as the PAM co-addition process. This PAM co-addition process changes the typical color of the mother liquor of 5-amino-N,N'-bis(2,3-dihydroxypropyl)-2,4,6-triiodoisophthalamide from red to yellow as well as giving superior visual and spectroscopic results.

The initial purpose of working in this field of study was to improve the standard co-addition process disclosed in U.S. Patent Number 5,013,865. An improvement was deemed necessary since the 5-amino-N,N'-bis(2,3-dihydroxypropyl)-2,4,6-triiodoisophthalamide made by the standard co-addition process resulted in ioversol with a slight yellow appearance. The final product color from the standard co-addition process led to the decision to improve the cosmetic appearance of the product. Accordingly, the standard co-addition process was to be analyzed and controlled in some way to limit the production of colored impurities in ioversol.

The exact nature of the colored impurity that gives rise to the yellow color in ioversol is related to the "azo" family of compounds formed from the oxidation of the substrate by iodine monochloride or its decomposition products. The azo compound from 5-amino-N,N'-bis(2,3-dihydroxypropyl)-2,4,6-triiodo-isophthalamide had previously been found in the methanol wash of the chromatography purification column and subsequently in the mother liquor from 5-amino-N,N'-bis(2,3-dihydroxypropyl)-2,4,6-triiodoisophthalamide. None of the non-iodinated azo compounds were detected in either the 5-amino-N,N'-bis(2,3-dihydroxypropyl)-2,4,6-triiodoisophthalamide mother liquor or solids. Accordingly, efforts were made to analyze and somehow control the amount of azo-bis(5,5'-N,N'-bis(2,3-dihydroxypropyl)-2,4,6-triiodo)-1,3-benzenedicarboxamide produced during the co-addition process.

During the analysis, the former standard co-addition process was found to produce a wide range of colored materials when initially investigated. From this, conditions were identified that led to gross color formation. Once identified, those conditions were avoided and the product 5-amino-N,N'-bis(2,3-dihydroxypropyl)-2,4,6-triiodoisophthalamide typically had good color. (Color evaluation was performed strictly on visual appearance of the dried product.) The conditions identified as desirable to produce ioversol of good color, i.e., white, were good agitation, moderate pH within the range of 0 to 3.0, and an iodine monochloride (ICl) flow lagging the buffered reduced amide (RDA) flow. A seven percent lag in the ICl flow was found to be satisfactory although as much as fifty percent seemed to be tolerable with good color results.

In the past, proper agitation of the aqueous reaction medium, i.e., water or a dilute acid, was believed to be achievable using standard reactor and impeller geometries operated at 125 rpm. Using this method, early batches of 5-amino-N,N'-bis(2,3-dihydroxypropyl)-2,4,6-triiodoisophthalamide had strong red color when processed through to crude ioversol. The agitation in the iodinators showed that the coloration developed in part because the original agitation configuration provided essentially solid body rotation and little mixing even at higher speeds. This undesirable result was due to high blade pitch (60°) of the agitator means, essentially no baffling low in the tank, and the high impeller rotation speed (125 rpm). Accordingly, through testing, it was determined that a 45° blade pitch and a slower, e.g., 75 rpm agitator speed resulted in better mixing with less solid body rotation. With the altered agitation, the measured color of the 5-amino-N,N'-bis(2,3-dihydroxypropyl)-2,4,6-triiodoisophthalamide at 450 nm decreased dramatically, by a factor of two to five. Another mixing scheme which gave approximately equal color results had a 30° blade pitch operated at 125 rpm.

The ioversol intermediates produced from the PAM co-addition process and the altered agitation process were now yellow in color whereas in the past, most of the intermediates produced by the former standard co-addition process and former agitation process were red.

An additional agitation study using water at various levels in the iodinators was also performed. At the lower fill levels of approximately six hundred (600) gallons, 125 rpm and 30° blade pitch was found to give the best axial flow pattern. Since the axial flow component is often missing in poorly mixed systems, this configuration looked very promising. However, at one thousand four hundred (1400) gallon fill levels corresponding to end of reaction levels, the mixing seemed to become poorer. The next best configuration was with a 45° blade pitch and 75 rpm. The mixing from this arrangement rapidly got worse as the fill level of the water within the iodinators increased from the initial six hundred (600) gallon charge. Increasing the rpm of the agitator however was found to improve the mixing at higher fill levels of one thousand (1000) gallons or more. At high fill levels of approximately one thousand four hundred (1400) gallons and 125 rpm the 45° pitch performed in the process similarly as that of low levels of approximately six hundred (600) gallons and 75 rpm. The ability to maintain similar mixing capability with increasing fill quantities led to the novel utilization of a ramped speed increase in the agitation, coordinated with the increasing fill level. The actual agitator speed was increased with time in coordination with the fill level which was also increased with time hence they were increased together. The agitation speed and fill volumes were effectively coordinated to increase at the same rate as is the preferred method to decrease color formation in the ioversol produced. Accordingly, depending upon the fill volume level and the agitator means blade pitch, proper agitation can be accomplished at speeds between the range of about 30 and 250 revolutions per minute.

Higher pHs were also found to have a large effect on ioversol colorization. Higher pHs, such as around 3 or greater, for the entire standard co-addition process produced strongly colored 5-amino-N,N'-bis(2,3 -dihydroxypropyl) -2,4,6-triiodoisophthalamide. Accordingly, it was decided to try to improve the former standard co-addition process by forcing the initial pH of the co-addition process to the same as that of the co-addition process during the majority of the reaction, i.e. 1.0 to 2.0 but preferably 1.5 to 2.0 rather than 2.5 to 3.0. Hydrochloric acid (HCl) was chosen to alter the pH since it is used in other steps in the synthesis of ioversol. A sufficient quantity of HCl was added to lower the initial water charge to a pH between 1.0 and 2.0 but preferably 1.5 to 2.0 which is the typical final pH range for the standard co-addition. The addition of hydrochloric acid to the initial water charge in the iodinator was not a risky change since HCl is actually generated during the reaction prior to being neutralized by the buffer. This new process was referred to as hydrochloric acid modified (HAM) co-addition. The HAM co-addition process was found to produce less iodine "fuming" early in the reaction. The reduced fuming was due to less ICl decomposition at that point in the reaction as is desired to decrease color formation. The HAM co-addition with the new ramped agitation process produced another 7% reduction in the measured color of the 5-amino-N,N'-bis(2,3-dihydroxypropyl)-2,4,6-triiodoisophthalamide. Dry 5-amino-N,N'-bis(2,3-dihydroxypropyl)-2,4,6-triiodo-isophthalamide color measured at 450 nm ranged from 0.025 AU to 0.035 AU. However, still further improvement in the reduction of color was needed in order to provide customer satisfaction with regard to the color of the ioversol produced.

Hydrochloric acid, as well as other acids, is known to stabilize ICl towards decomposition by tending to convert H₂OI⁺ to ICl. It is for this reason that in order to achieve further stabilization HCl and some NaCl historically have been added to the ICl makeup. However, the use of HCl in the 5-amino-N,N'-bis(2,3-dihydroxypropyl)-2,4,6-triiodoisophthalamide iodination process was not as effective at reducing the color of the final product as expected. The iodine fumes noted during the reaction were reduced significantly indicating that the decomposition of ICl was slowed as expected. Therefore, it could be argued that the addition of more HCl would have solved the remaining colorization problem. Unfortunately, the addition of a sufficient quantity of HCl to reduce the color of the ioversol to the point desired would inhibit the iodination reaction to the point that the HAM co-addition would be no better than the former standard co-addition process.

Although it was believed that phosphoric acid would likewise inhibit the iodination reaction as experienced by the hydrochloric acid an effort was made to test the theory. Accordingly, phosphoric acid (H₃PO₄) was added in the initial water charge of the reaction, even though hydrochloric acid (HCl) was unsuccessful. The decrease in pH caused by the addition of H₃PO₄ was hoped to stabilize the ICl. The H₃PO₄ was added to the co-addition process just as the buffer, i.e. sodium hydroxide and phosphate, neutralized the HCl naturally produced by the reaction. Since the HCl would be neutralized by the buffer, it was believed that phosphate ions would then be present to catalyze the iodination reaction. A major chemical concern with the use of phosphoric acid is the role of the phosphate ion in catalyzing the hydrolysis reaction of ICl. At lower pHs, the presence of a high concentration of H⁺ retards the hydrolysis of ICl as was experienced with the HAM co-addition process. However, surprisingly the iodination reaction was not inhibited by the PAM co-addition process as seen in the HAM co-addition process.

The specific details of the phosphoric acid modified (PAM) co-addition process of the present invention for use in the production of ioversol and like iodinated contrast agents are described in and may be carried out in accordance with the following specific examples:

### EXAMPLE 1:

Reduced diamide and iodine monochloride were used after being assayed. Charges were based on the assay. Phosphoric acid, 75%, and phosphate buffer were used as received without adjustment for strength. 50% sodium hydroxide was for the most part used as received. Sodium bisulfite and other analytical reagents were likewise used as received. All deionized water was used throughout the process described.

A convenient scale for the lab was determined to be 150 mmole reduced diamide. The phosphate buffer and ICl amounts were proportioned to this scale. Reactions were run in a 4 neck, 1 liter, round bottom flask. The flask was equipped with an overhead stirrer, a reflux condenser, thermometer and temperature controller, pH electrode, and a baffle with two addition lines to introduce chemicals into the flask. Temperature control was provided using a Thermowatch® (manufactured by Instruments for Industry Research, Cheltenham, PA) connected to a variable transformer and a heating mantle. The thermometer and reflux condenser were on the same neck through the use of a Claisen adapter. The pH electrode was located two hundred seventy degrees around the flask in the direction of rotation from the addition lines. The addition lines were arranged in such a manner that the ICl was injected subsurface, counter current to the direction of flow of the bulk solution. The buffered reduced diamide (RDA) and sodium hydroxide used to adjust the final pH were added above the surface. The ICl and buffered reduced diamide lines were oriented in such a manner that the solutions discharged in opposite directions. An initial charge of 300 ml deionized water was added to the flask. 75% phosphoric acid was added to the water charge. The RDA was measured out on the basis of the assay and 84 ml of the previously prepared buffer were added, mixed, and the total volume was determined. ICl was measured out on the basis of its assay. The stirrer was turned on and the initial charge was heated to 80°C; then the PAM co-addition was begun. Flow control was provided for the buffered reduced diamide (RDA) and ICl by Masterflex® pumps (manufactured by Cole Parmer, Niles, IL). Water was used to set the pumping rate for each solution. The rate was determined by the ratio of the volume of reagent to the desired addition time. The flow rate was monitored by checking the amount that had been added over continuous five minute intervals. Small adjustments were made in order to maintain the proper flow rates and ratio. Samples were taken at the same five minute intervals to profile the extent of reaction. The extent of the reaction was determined by high performance liquid chromatography (HPLC) peak area percent of the reaction mixture. Previous work showed the reactions to be complete ninety minutes after the end of addition. Samples were taken less frequently during the ninety minute hold time. After the hold period was completed, cooling to 60°C was begun and sodium bisulfite was added to quench any excess ICl remaining. The sodium bisulfite also quenched any I₂ formed during the course of the reaction. Enough sodium bisulfite was added so that no free iodine was observed in the upper portions of the reaction flask and starch-iodide paper no longer gave a positive test. Sodium hydroxide was added to adjust the pH of the solution to a range between 5.5 and 7. A small amount of seed crystals was added and the reaction mixture was held at 60°C for 1 hour. The reaction mixture was then allowed to cool to room temperature, usually overnight. The crystals were allowed to settle and the supernatant liquid was decanted to a 9 cm Buchner funnel with #41 paper on a vacuum flask. The crystals were then transferred to the filter and were washed with a minimum amount of deionized water. The crystals were allowed to air dry on the filter by drawing air over them.

A baseline standard co-addition reaction was also run profiling the reaction products with time. This baseline standard co-addition reaction was used to give a starting point of what the former standard co-addition process was like.

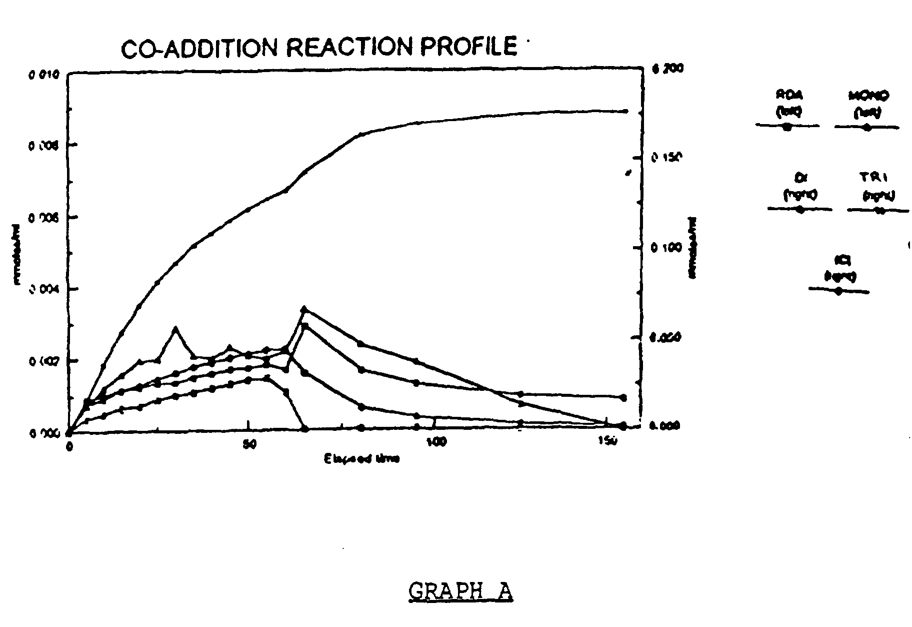

As can be seen in the above graph, there is virtually no reason to run the reaction more than ninety minutes beyond the end of addition. In fact, considering that the only color body thus far isolated was azo-bis(5,5'N,N'bis(2,3-dihydroxypropyl)-2,4,6-triiodo)-1,3-benzenedicarboxamide, one might argue that extending the reaction time beyond that point may lead to more azo impurities being formed. The concentrations of the organic species were calculated from the high performance liquid chromatography (HPLC) process. The ICl concentration was calculated from the difference of the amount added and the amount consumed to make the iodinated organic species. The appearance of the graph was normal with the RDA having an early spike and the ICl having a late spike due to the relative differences in the flow rates. The reference to right and left in the legend refers to the right or left axis which differ by a factor of ten.

The first PAM co-addition process reaction had an initial charge of phosphoric acid, 6.2 ml, which was sufficient to reduce the pH of the water to 1.5, comparable to the better pH conditions for the unsuccessful HAM co-addition process. The isolated yield of crude, first crop 5-amino-N,N'-bis(2,3-dihydroxypropyl)-2,4,6-triiodoisophthalamide was surprisingly over 87%. Also to the inventor's surprise, iodine fuming was virtually eliminated. Plotting the reaction profile from the HPLC process data showed that the concentration spike of RDA at the beginning of the reaction was eliminated and that conversion to 5-amino-N,N'-bis(2,3-dihydroxypropyl)-2,4,6-triiodo-isophthalamide was unexpectedly faster than a normal, i.e., the standard, co-addition process. The mother liquor had very little of the red color that was previously also associated with the standard co-addition. Both of these reactions and all subsequent ones were run using a 1% excess of ICl. The color of the mother liquor seemed to be a good indicator of the effectiveness of the ability of the phosphoric acid to reduce the formation of color bodies.

A similar study was likewise conducted, in which the pH was lowered to 1 in the water charge of the addition process which produced no RDA concentration spike at the beginning of the reaction and produced a yellow mother liquor; 12 ml of phosphoric acid were required to get the desired pH of 1. All subsequent reactions were then performed using a fixed phosphoric acid charge of 12 ml regardless of the pH found in the solution.

Still another improvement of the present invention for reducing color formation in the 5-amino-N,N'-bis(2,3-dihydroxypropyl)-2,4,6-triiodo-isophthalamide, was to reduce the spike in ICl concentration that occurs due to the lag in ICl feed as compared to RDA in the reaction process. There are two ways to accomplish the ICl concentration spike reduction. The first way was accomplished by reducing the addition rate of ICl after all the RDA was added. This would still lead to a spike, but at least one of lower amplitude. The overall rate to 5-amino-N,N'-bis(2,3-dihydroxypropyl)-2,4,6-triiodoisophthalamide was also expected to be lower with the addition rate of ICl decreased. The second way to eliminate the spike was accomplished by increasing the ICl flow to the same addition time as that of the RDA. Since the addition of phosphoric acid to the reaction seemed to reduce the decomposition of ICl dramatically, it appeared feasible to increase the ICl flow rate. Accordingly, a reaction was run with a faster ICl flow rate. The resulting reaction profile had no ICl spike and more rapid conversion to 5-amino-N,N'-bis(2,3-dihydroxypropyl)-2,4,6-triiodoisophthalamide than the other reactions conducted as preferred to avoid color formation. Unexpectedly, the final yield was greater than 95% and the mother liquor color was similar to the previous desirable PAM co-addition reactions. (The initial isolated yield was 87% for the crude with no second crop recovery.)

Also interesting to note is that the peak created by the mono-iodinated species concentration appeared about halfway through the addition process. This spike was observed for many phosphoric acid modified co-addition reactions. The reason for the spike is not known but is speculated to be caused by the minor adjustments made in addition rate to maintain an overall correct addition time. The spike does not appear to be an artifact of the calculation of the monoiodo peak.

The sensitivity of the PAM co-addition process to mixing was then tested in two reactions with poorer mixing than previously attempted, 50 versus 250 rpm. One with RDA and ICl addition rates nearly equal and the other with the normal 7% lag in the ICl addition rate. In both cases, about halfway through the addition the quenched reaction solution samples changed color from yellow (as was seen in the higher agitation speed experiments) to an orange color. The final mother liquor color was a similar orange color. The other data collected showed no difference between the two systems. This would suggest that there is a fairly strong dependence of color formation on the agitation of the reaction. The samples were later retested for the presence of azo-bis(5,5'-N,N'-bis(2,3-dihydroxypropyl)-2,4,6-triiodo)-1,3-benzenedicarboxamide. The following Graph B shows the relative amount of azo present. Quantification was not possible with these samples. It appears that the relative amount of azo increases with time. This should not be construed to mean anything directly regarding the decomposition of ICl which is necessary to form azo.

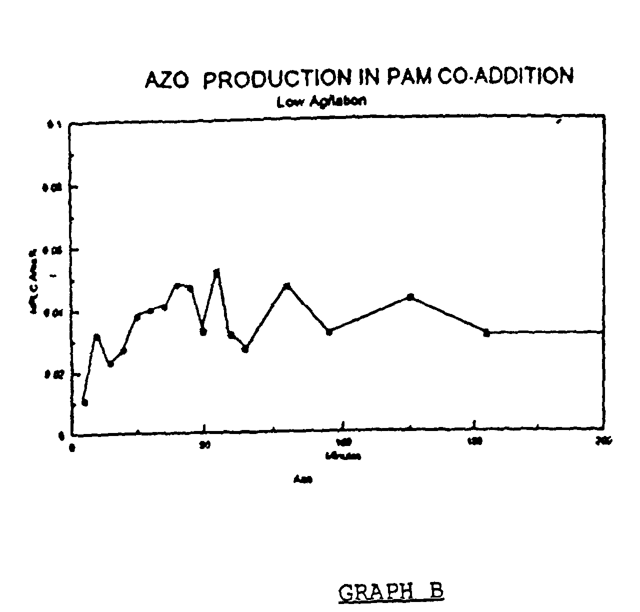

Two reactions were run to test the effect of having no buffer present on the standard co-addition reaction. Strong iodine fuming was noted and the color of the unquenched reaction solution was darker than with the PAM process. In each case, the color of the quenched reaction solution was yellow during the course of the reaction, but after quenching the bulk of the reaction with bisulfite, the mother liquor color was green. There also appeared to be a greenish cast to the final product. A green color had not been observed in iodination before. No azo or hydrazo impurities were found in the HPLC of the mother liquor. The reaction profile showed the normally expected shape for an uncatalyzed reaction. The yield was lower than in either buffered standard co-addition or PAM reactions.

The reaction having no buffer added runs at a much lower pH (1 to <0) than does either the standard co-addition process (2 to 1.5) or the PAM co-addition process (1.5 to 1) reactions. A much higher concentration of RDA was also observed throughout the course of the "no buffer" reaction, probably due to the formation of more H⁺RDA, which must then dissociate in order to iodinate.

Several repeat reactions were run in order to obtain sufficient 5-amino-N,N'-bis(2,3-dihydroxypropyl)-2,4,6-triiodoisophthalamide to prepare enough ioversol on the lab scale for evaluation. In general the reactions went well. In two instances, the buffered RDA line plugged, but no major imbalance between the feed ratio of ICl and RDA occurred. These reactions finished with the yellow mother liquor which had become indicative of a good PAM reaction.

A yellow color was noted in the distillate when the PAM ioversol hexaacetate was hydrolyzed. The color was determined to come from iodine. There was no obvious reason why iodine should be evolved at this step. The ioversol, after purification, was evaluated both visually and spectrophotometrically and found to be superior to that derived from the standard co-addition process.

The inclusion of phosphoric acid in the initial water charge of the co-addition process to make 5-amino-N,N'-bis(2,3-dihydroxypropyl)-2,4,6-triiodo-isophthalamide has thus been shown to be unexpectedly beneficial in shifting the color of the mother liquor from red to yellow and in increasing yields of the iodinated intermediate. Testing of the mother liquor showed much lower levels of azo-bis(5,5'-N,N'-bis(2,3-dihydroxypropyl)-2,4,6-triiodo)-1,3-benzenedicarboxamide in the PAM mother liquors than was found in buffered standard co-addition mother liquors. The PAM 5-amino-N,N'-bis(2,3-dihydroxypropyl)-2,4,6-triiodoisophthalamide processed through to ioversol gave acceptable color in the final ioversol product. Some agitation dependency was noted in the mother liquor color and the amount of azo-bis-5,5'-N,N'-bis(2,3-dihydroxypropyl)-2,4,6-triiodo)-1,3-benzenedicarboxamide produced.

It is important to note that commonly available concentrations of the H₃PO₄ and NaOH may be used in the above described invention, i.e., 75-85% and 30-50% respectively.

As various changes could be made in the above process without departing from the scope of the present invention, it is intended that all matter contained in the above description or shown in the above examples shall be interpreted as illustrative and not in a limiting sense.

## Claims

1. A process for the preparation of an iodinated product compound comprising reacting a substrate with an iodine halide in an aqueous reaction medium wherein said iodine halide is in a stoichiometric excess over said substrate, **characterised in that** phosphoric acid or phosphoric acid and a salt thereof are added to the aqueous reaction medium prior to the addition of the substrate and the iodine halide.

2. A process for the preparation of an iodinated product compound comprising reacting a substrate with an iodine halide in an aqueous reaction medium wherein said iodine halide is in a stoichiometric excess over said substrate, **characterised in that** phosphoric acid or phosphoric acid and a salt thereof are added to the aqueous reaction medium prior to the addition of the substrate and the iodine halide, and agitating said aqueous reaction medium in the presence of baffling means and at a rate capable of providing mixture of said aqueous reaction mixture rather than solid body rotation.

3. The process of claim 1 or 2 wherein the phosphoric acid or phosphoric acid and a salt thereof are added to the aqueous reaction medium such that the initial pH is between 1.0 to 2.0.

4. The process of any preceding claim, wherein said substrate is 5-amino- N,N'-bis(2,3-dihydroxypropyl) isophthalamide.

5. The process of any preceding claim wherein said iodine halide is iodine monochloride solution.

6. The process of any one of claims 1 to 4 wherein said iodine halide is aqueous sodium dichloroiodate.

7. The process of any preceding claim wherein said aqueous reaction medium is water.

8. The process of any one of claims 1 to 6 wherein said aqueous reaction medium is a dilute acid.

9. The process of claim 2 wherein said agitation is accomplished with agitation means rotated at a speed between the range of 30 and 250 revolutions per minute.

10. The process of any preceding claim wherein said iodine halide is introduced into said aqueous reaction medium at a 7 to 50 percent lag in flow rate to that of said substrate flow.

## Patentansprüche

1. Verfahren zur Herstellung einer iodierten Produktverbindung, umfassend das Umsetzen eines Substrats mit einem lodhalogenid in einem wässerigen Reaktionsmedium, wobei das lodhalogenid in einem stöchiometrischen Überschuß gegenüber dem Substrat vorliegt, **dadurch gekennzeichnet, daß** Phosphorsäure oder Phosphorsäure und ein Salz davon zu dem wässerigen Reaktionsmedium vor der Zugabe des Substrats und des lodhalogenids zugegeben wird/werden.

2. Verfahren zur Herstellung einer iodierten Produktverbindung, umfassend das Umsetzen eines Substrats mit einem lodhalogenid in einem wässerigen Reaktionsmedium, wobei das lodhalogenid in einem stöchiometrischen Überschuß gegenüber dem Substrat vorliegt, **dadurch gekennzeichnet, daß** Phosphorsäure oder Phosphorsäure und ein Salz davon zum wässerigen Reaktionsmedium vor der Zugabe des Substrats und des lodhalogenids zugegeben wird/werden, und das Rühren des wässerigen Reaktionsmediums in Gegenwart von Ablenkmitteln und bei einer Geschwindigkeit, welche eher zur Bereitstellung einer Vermischung des wässerigen Reaktionsgemischs als einer Festkörperrotation befähigt ist.

3. Verfahren nach Anspruch 1 oder 2, wobei die Phosphorsäure oder Phosphorsäure und ein Salz davon derart zum wässerigen Reaktionsmedium zugegeben wird/werden, daß der anfängliche pH-Wert zwischen 1,0 bis 2,0 liegt.

4. Verfahren nach einem der vorhergehenden Ansprüche, wobei das Substrat 5-Amino-N,N'-bis(2,3-dihydroxypropyl)isophthalamid ist.

5. Verfahren nach einem der vorhergehenden Ansprüche, wobei das lodhalogenid eine lodmonochloridlösung ist.

6. Verfahren nach einem der Ansprüche 1 bis 4, wobei das lodhalogenid wässeriges Natriumdichloriodat ist.

7. Verfahren nach einem der vorhergehenden Ansprüche, wobei das wässerige Reaktionsmedium Wasser ist.

8. Verfahren nach einem der Ansprüche 1 bis 6, wobei das wässerige Reaktionsmedium eine verdünnte Säure ist.

9. Verfahren nach Anspruch 2, wobei das Rühren mit Rührmitteln durchgeführt mit, welche bei einer Geschwindigkeit im Bereich zwischen 30 und 250 Umdrehungen pro Minute rotieren.

10. Verfahren nach einem der vorhergehenden Ansprüche, wobei das lodhalogenid in das wässerige Reaktionsmedium bei einer 7 bis 50 Prozent geringeren Flußrate im Vergleich zum Fluß des Substrats eingebracht wird.

## Revendications

1. Procédé de préparation d'un composé produit iodé comportant la réaction d'un substrat avec un halogénure d'iode dans un milieu réactionnel aqueux dans lequel ledit halogénure d'iode est en excès stoechiométrique par rapport audit substrat, **caractérisé en ce que** de l'acide phosphorique ou de l'acide phosphorique et un de ses sels sont ajoutés au milieu réactionnel aqueux avant l'ajout du substrat et de l'halogénure d'iode.

2. Procédé de préparation d'un composé produit iodé comportant la réaction d'un substrat avec un halogénure d'iode dans un milieu réactionnel aqueux dans lequel ledit halogénure d'iode est en excès stoechiométrique par rapport audit substrat, **caractérisé en ce que** de l'acide phosphorique ou de l'acide phosphorique et un de ses sels sont ajoutés au milieu réactionnel aqueux avant l'ajout du substrat et de l'halogénure d'iode, et l'agitation dudit milieu réactionnel aqueux en présence de moyens de chicanage et à une vitesse capable d'assurer le mélange dudit milieu réactionnel aqueux plutôt qu'une rotation des corps solides.

3. Procédé de la revendication 1 ou 2 dans lequel l'acide phosphorique ou l'acide phosphorique et un de ses sels sont ajoutés au milieu réactionnel aqueux de façon que le pH initial soit compris entre 1,0 et 2,0.

4. Procédé de l'une quelconque des revendications précédentes, dans lequel ledit substrat est le 5-amino-N,N'-bis(2,3-dihydroxypropyl)isophtalamide.

5. Procédé de l'une quelconque des revendications précédentes dans lequel ledit halogénure d'iode est une solution de monochlorure d'iode.

6. Procédé de l'une quelconque des revendications 1 à 4 dans lequel ledit halogénure d'iode est du dichloroiodate de sodium aqueux.

7. Procédé de l'une quelconque des revendications précédentes dans lequel ledit milieu réactionnel aqueux est de l'eau.

8. Procédé de l'une quelconque des revendications 1 à 6 dans lequel ledit milieu réactionnel aqueux est un acide dilué.

9. Procédé de la revendication 2 dans lequel ladite agitation est accomplie avec des moyens d'agitation tournant à une vitesse comprise dans la gamme de 30 à 250 tours par minute.

10. Procédé de l'une quelconque des revendications précédentes dans lequel ledit halogénure d'iode est introduit dans ledit milieu réactionnel aqueux avec un retard de 7 à 50 % dans le débit par rapport à celui dudit courant de substrat.
